# EUROPÄISCHE PATENTANMELDUNG

(11) **EP 1 681 055 A1**
(43) Veröffentlichungstag der Anmeldung: **19.07.2006**
(21) Anmeldenummer: 05000596.6
(22) Anmeldetag: 13.01.2005
(51) Int. Cl.: A61K 31/4415, A61K 31/198, A61P 9/10

(54) **Pharmazeutische Zubereitung zur Behandlung eines erhöhten Homocysteinspiegels**

(71) Anmelder: PEJO Iserlohn Heilmittel-und Diät-GmbH & Co.KG, 58638 Iserlohn (DE)
(72) Erfinder: Pütter, Sigurd, 58638 Iserlohn (DE); Kalisch, Peter, 58708 Menden (DE); Ammer, Richard, 58638 Iserlohn (DE); Riezler, Reiner, 48163 Münster (DE)
(74) Vertreter: Reinhard - Skuhra - Weise & Partner

(57) **Zusammenfassung**

Die vorliegende Erfindung beschreibt eine pharmazeutische Zusammensetzung zur Behandlung erhöhter Homocysteinspiegel, enthaltend eine therapeutisch wirksame Menge an Vitamin B6 und Acetylcystein, und/oder ihrer physiologisch akzeptablen Derivate und/oder ihrer Vorstufen und/oder ihrer pharmazeutisch wirksamen Salze, zusammen mit pharmazeutisch üblichen Träger- und/oder Hilfsstoffen. Als weitere Wirkstoffe sind bevorzugt Vitamin B12 und/oder Folsäure enthalten.

## Beschreibung

Die vorliegende Erfindung betrifft ein phannazeutische Zusammensetzung zur Behandlung erhöhter Homocysteinspiegel, ein Verfahren zur Herstellung dieser Zusammensetzung sowie die Verwendung der Zusammensetzung zur Therapie und Prophylaxe erhöhter Homocysteinwerte.

Kardiovaskuläre Erkrankungen sind sowohl in der allgemeinen Bevölkerung als auch bei Patienten mit terminaler Niereninsuffizienz die Haupttodesursache. Gegenüber der allgemeinen Bevölkerung haben die Patienten unter terminaler Niereninsuffizienz ein 5- bis 20-fach höheres Risiko, an einer Herz-Kreislauf-Erkrankung zu sterben. Klinische Studien belegen, dass die Schädigung der Gefäße für das erhöhte Risiko bei Patienten mit terminaler Niereninsuffizienz verantwortlich ist. Gefäßverkalkungen und eine Abnahme der Gefäßelastizität sind Indikatoren für ein erhöhtes kardiovaskuläres Risiko.

Ein erhöhter Wert an Homocystein wird in verstärktem Maße als Risikofaktor für periphere arterielle Gefäßerkrankungen und cerebrale Gefäßerkrankungen angesehen. Neuere Studien (15,16,17) zeigen, dass das erfolgreiche Absenken des Homocysteins auf Werte < 9µmol/l mit einer Kombination aus B6, B12 und Folsäure die Notwendigkeit eine erneute Revaskularisierung bei Patienten mit koronarer Gefäßerkrankung nach einer Angiographie reduziert.

Eine effektive Absenkung des Homocysteins konnte auch durch i.v. Gabe von B6, B 12 und Folsäure in klinischen Studien (unveröffentlichte Daten) gezeigt werden.

Homocystein tritt als zellgängige Verbindung im Blut auf und liegt dort in verschieden Formen vor: als freie Aminosäure, als Thiolacton, als Homocystin, als gemischtes Dimer mit Cystin und als Dipeptid aus Homocystein-Thiolacton und Homocystein. Ca. 70% des Homocysteins liegen als Disulfid oder peptidisch gebunden an Plasma oder Lipoproteine gebunden vor. Es steht am Kreuzungspunkt zweier metabolischer Kreisläufe: der Remethylierung und der Transsulphurierung.

Acetylcystein wird gut in die Zelle aufgenommen und zu Glutamathion umgebaut. ACC fungiert aber auch als SH-Gruppenspalter (schleimlösender Effekt). Daher setzt es wahrscheinlich das Homocystein aus der "schützenden" Lipidbindung frei, wodurch Homocystein in seinem negativen Einfluss auf das Gefäßendothel weiter an Wirkung gewinnt (13).

Gerade bei Dialysepatienten wurden erhöhte Homocysteinspiegel beobachtet, die zu einer erhöhten Rate an kardiovaskulären Erkrankungen und Todesfällen führen. Die Ursachen und bisherigen Therapiemethoden werden nachfolgend näher diskutiert.

Die kardiovaskuläre Mortalität von Dialysepatienten ist in jeder Altersgruppe gegenüber der Allgemeinbevölkerung drastisch erhöht. Die Ursachen hierfür liegen unter anderem in einer multifaktoriell bedingten akzelerierten Arteriosklerose. Einen bedeutenden Faktor hierfür stellt die Hyperhomozysteinämie (HHCY) dar, die wie in der Allgemeinbevölkerung, auch bei Patienten mit dialysepflichtiger chronischer Niereninsuffizienz als kardio- und zerebrovaskulärer Risikofaktor betrachtet wird (1-3). Eine Erhöhung der Homozysteinspiegel über den Normbereich hinaus findet sich fast bei jedem chronischen Hämodialysepatienten.

Der einer HHCY bei chronischer Niereninsuffizienz zugrundeliegende Pathomechanismus ist zwar noch unzureichend bekannt, wird jedoch zumindest zum Teil durch eine verminderte renale Clearance und Metabolisierung erklärt (4,5). Der zytotoxische Effekt einer HHCY beruht auf der Induktion von oxidativem Stress, einer Veränderung der Endothelzell- und Plättchenfunktion mit prothrombotischer Wirkung sowie einer Störung der NO-Produktion (6,7).

Die derzeit verfügbaren diskontinuierlichen Dialyseverfahren sind nicht in der Lage, die Nierenfunktion hinsichtlich des Managements von Homozystein (HCY) adäquat zu ersetzen. Da die Elimination von HCY durch die Hämodialyse unzureichend ist, müssen Methoden zur effektiven medikamentösen Senkung der HCY-Spiegel entwickelt werden. Während es bei nierengesunden Patienten durchaus möglich ist, HCY-Spiegel durch die Gabe von Vitamin B6, Vitamin B 12 und Folsäure adäquat zu senken (5), zeichnen sich Dialysepatienten durch eine unzureichende Wirkung dieses Therapieansatzes aus (8,9). Zwar wurden in Studien mit Vitamin B Substitution bei chronischen Dialysepatienten markante Senkungen der HCY-Spiegel erreicht, zu einer Normalisierung der HCY-Werte kam es allerdings nur selten (10,11).

Selbst orale supraphysiologische Dosen von Folsäure und B-Vitaminen führten nur bei einem kleinen Prozentsatz von Dialysepatienten zum Ziel (12).

N-Acetyl-Cystein (NAC) führt bei Gesunden zu einer Steigerung der säurelöslichen, nicht protein-gebundenen und somit renal eliminierbaren Fraktion von HCY (13). Bei Dialysepatienten könnte die Steigerung der nicht protein-gebundenen Fraktion somit zu einer Verbesserung der Elimination von HCY während der Dialyse führen. Diese Hypothese ist kürzlich untersucht worden (14). Die Verabreichung von 1200 mg NAC vor einer Dialysebehandlung führte dabei zu keiner Steigerung der HCY-Elimination während der Dialyse, wohl aber fand sich eine im Mittel 16-prozentige Verminderung der prä-dialytischen Serum-Werte von HCY im Vergleich zu einem Dialysetag ohne vorherige Einnahme von NAC. Die HCY-senkende Wirkung von NAC zeigte sich innerhalb relativ kurzer Zeit, aber der HCY Spiegel kehrte nach Absetzen auch wieder innerhalb von Tagen zum Ausgangswert zurück.

Bei Gabe von ACC allein konnte an gesunden Probanden zwar ein Absenken des Gesamthomocysteins gezeigt werden, dafür wurde aber die Konzentration des freien lipidungebundenen Homocysteins, dem die Schädigung des Gefäßendothels zugeschrieben wird, erhöht.

Herrmann et al. (18) konnte durch Gabe von B6 keinen Einfluss auf den Homocysteinspiegel zeigen. Die Gabe von ACC allein bewirkt nach diesen Autoren nur ein geringes Absenken des Homocysteins.

Es ist eine Aufgabe der vorliegenden Erfindung, eine neue pharmazeutische Zusammensetzung bereitzustellen, welche den Transsulphurierungsweg öffnet und hierdurch erhöhte Homocysteinwerte, insbesondere auch bei Dialysepatienten, effizient absenkt, eine gute Verträglichkeit und Bioverfügbarkeit aufweist und die auch in der Pädiatrie ohne gravierende Nebenwirkungen einsetzbar ist.

Diese Aufgabe wird erfindungsgemäß gelöst durch eine pharmazeutische Zusammensetzung, enthaltend eine therapeutisch wirksame Menge an Vitamin B6 und Acetylcystein, und/oder ihrer physiologisch akzeptablen Derivate und/oder ihrer Vorstufen und/oder ihrer pharmazeutisch wirksamen Salze, zusammen mit pharmazeutisch üblichen Träger- und/oder Hilfsstoffen.

Erfindungsgemäß wurde überraschend festgestellt, daß eine effiziente Öffnung des Transsulphurierungsweges durch die kombinierte Verabreichung von Vitamin B6 und Acetylcystein ermöglicht wird. Hierdurch wird Homocystein in außerordentlich hohem Maße zu Cystathionin und Cystein abgebaut. Cystein wird über Gamma-Glutamincystein zu Glutathion verstoffwechselt, welches als Zellaktivator wirkt und die antioxidative Kapazität der Zelle verbessert. Durch die erfindungsgemäße pharmazeutische Zusammensetzung wird also nicht nur eine wirksame Absenkung des Gesamt-Homocysteinspiegels bewirkt, sondern gleichzeitig werden auch die Cystathionin- und Cysteinwerte effizient abgesenkt und die Glutathionspiegel angehoben. Gerade dem Glutathion kommen antioxidative Fähigkeiten zu, sodass eine zusätzliche zellprotektive Wirkung einsetzt. Hierdurch werden die Zellwände und der Patient unter anderem vor kardiovaskulären Erkrankungen geschützt. Im Mittelpunkt der vorliegenden Erfindung steht deshalb nicht nur eine Absenkung des Homocysteinspiegels, sondern insbesondere die unerwartet effiziente Öffnung des Transsulphurierungsweges, die unerwartet deutliche Absenkung der Cystathionin- und Cysteinspiegel und die Erhöhung des Glutathionspiegels. Alle diese durch die erfindungsgemäße Zusammensetzung erreichten Effekte führen zu den erwünschten zellprotektiven prophylaktischen und therapeutischen Wirkungen für den Patienten.

Während bei einer Verabreichung von Homocystein als alleinigen Wirkstoff und von Acetylcystein als alleinigen Wirkstoff nur eine geringe Absenkung des Gesamt-Homocysteinspiegels möglich war, konnte unerwarteterweise durch eine kombinierte Verabreichung von Vitamin B6 und Acetylcystein ein überadditiver Effekt in der Homocysteinabsenkung beobachtet werden. Weiterhin konnte gezeigt werden, dass eine deutliche Tendenz zu normalen Cystathionin- und Cysteinspiegeln durch die kombinierte Gabe von Vitamin B6 und Acetylcystein erreichbar war, eine Wirkung, die weder durch die Verabreichung von Vitamin B6 noch durch die Verabreichung von Acetylcystein beobachtbar war.

Erfindungsgemäß wird also eine in dieser Deutlichkeit nicht erwartete Absenkung des Gesamt-Homocysteinspiegels erreicht. Insbesondere wichtig ist aber auch die beobachtete Wirkung, dass nicht nur der Homocysteinspiegel in Richtung normaler Werte abgesenkt werden konnte, sondern dass durch die gemeinsame Verabreichung beider Wirkstoffe der Transsulphurierungsweg erfolgreich und wirksam geöffnet wurde. Dies führt zu einer außerordentlich starken und nachhaltigen Absenkung des Cystathionin- und Cysteinspiegel und zu einer Erhöhung der Glutahtionwerte. Im Gegensatz zu anderen Homocysteinsenkern wie S-Adenosylmethionin wird der Homocysteinspiegel deutlich effizienter und über physiologische Abbaumechanismen abgesenkt.

Der Begriff "pharmazeutische Zusammensetzung bzw. Zubereitung", wie er vorliegend verwendet wird, ist nicht auf apothekenpflichtige oder gar verschreibungspflichtige Zubereitungsformen eingeschränkt. Er umfaßt vielmehr alle medizinisch wirksamen Zusammensetzungen, die beispielsweise auch in Form von Nahrungsergänzungsmitteln, aber auch Lebensmitteln, z.B. "functional food" oder diätetischen Lebensmitteln, angeboten werden können; diese dienen zur Ergänzung der täglichen Ernährung mit der erfindungsgemäßen Kombination aus Vitamin B6 und Acetylcystein.

Die erfindungsgemäße pharmazeutische Zusammensetzung kann auch weitere Wirkstoffe enthalten, die sich in sinnfälliger Weise mit den Wirkstoffen Vitamin B6 und Acetylcystein kombinieren lassen. Hierzu gehören insbesondere als weitere Vitamine B 12 und Folsäure. In einer bevorzugten Ausgestaltungsform der Erfindung liegen alle vier Wirkstoffe in Kombination miteinander vor.

Die erfindungsgemäße Zusammensetzung ist nicht nur zur Therapie einsetzbar, sondern auch zur Prophylaxe erhöhter Homocysteinspiegel und damit auch zur Prophylaxe von Vitaminmangelzuständen, Durchblutungsstörungen, kardio- und zerebrovaskulären Erkrankungen, Neuropathien und Kachexien.

Die oben näher bezeichneten Wirkstoffe werden bevorzugt in einer Applikationsdosis beziehungsweise Formulierung in Verbindung miteinander verabreicht. Es ist aber auch möglich, sie als "kits-of-parts"-Zusammensetzung zu formulieren, d.h. die Wirkstoffe können in zwei oder drei oder vier verschiedenen Formulierungen vorliegen, die dann entweder gemeinsam oder in einem zeitlichen Intervall, aber immer in therapeutisch enger Verbindung miteinander, verabreichbar sind.

Die Wirkstoffe in der pharmazeutischen Zusammensetzung können vom Fachmann in physiologisch akzeptablen und therapeutisch bzw. prophylaktisch wirksamen Mengenverhältnissen miteinander vermischt werden. Die zu formulierenden Mengen und die zu verabreichenden Dosen hängen von der Art der Applikationsform, von der Art der zu therapierenden Erkrankung und vom Zustand des Patienten (Alter, Größe, Geschlecht, Gewicht, Schwere der Erkrankung, Wechselwirkung mit anderen Medikamenten) ab. Die geeigneten Mengenverhältnisse können vom Fachmann anhand von Reihen- und Handversuchen ermittelt werden.

In bevorzugten Ausführungsformen der Erfindung liegt Vitamin B6 in einer Menge vor, so daß pro Tag 0,1 - 500 mg, bevorzugt 0,1 - 300 mg, weiterhin bevorzugt 5 - 200 mg oder 5 - 150 mg oder 5 - 100 mg appliziert werden. Besonders bevorzugt liegen die Vitamin B6-Mengen bei < 100 mg/Tag, bevorzugt bei 0,1 - 100 mg/Tag bzw. bis 150 mg/Tag.

Die Menge an pro Tag zu verabreichendem Acetylcystein in der erfindungsgemäßen Wirkstoffkombination liegt bei 0,1 - 5000 mg, bevorzugt 10 - 4000 mg, weiterhin bevorzugt 500 - 4000 mg oder 50 - 2500 mg. Insbesondere bevorzugt liegt die Acetylcysteinmenge bei ca. 1500 - 2000 mg/Tag, ganz besonders bevorzugt bei ca. 1800 mg. Weitere mögliche und bevorzugte Dosierungsbereiche liegen bei 0,1 - 3600 mg bzw. 0,1 - 1800/Tag.

Die Menge an Folsäure, die pro Tag verabreicht wird, liegt in einem Bereich von 0,1 - 30 mg, bevorzugt bis 25 mg, weiterhin bevorzugt im Bereich von 25 - 30 mg.

Vitamin B12 kann bevorzugt in einer Menge von 0,01 - 3 mg verabreicht werden, wobei Bereiche von 0,01 - 1,0 mg bzw. 2 - 3 mg besonders bevorzugt sind. Weiterhin bevorzugt ist eine Menge von ca. 0,5 mg B12 und ca. 5 mg Folsäure, jeweils pro Tag.

Die zu verabreichenden Einzeldosen können von einem Pharmazeuten in geeigneter Weise, je nach Applikationsform, ausgewählt werden. Besonders bevorzugt ist bei Acetylcystein eine Einzeldosis von ca. 300 - 600 mg, bei Vitamin B6 eine Einzeldosis von 50 - 100 mg. Bei Vitamin B6 hat sich bei vielen Patienten eine kritische Obergrenze von ca. 100 mg/Tag ergeben, da ansonsten die Gefahr von Neuropathien zunimmt.

Die maximalen Bereiche von Vitamin B6 liegen bei ca. 0,1 - 300 mg/Tag, bevorzugt bis 500 mg/Tag und insbesondere bevorzugt im Bereich von 0,1 - 100 mg/Tag.

Bei Acetylcystein liegen die Bereiche von ca. 0,1 - 3600 mg/Tag, besonders bevorzugt bei 0,1 - 1800 mg/Tag.

Die Mengenverhältnisse zwischen Vitamin B6 und Acetylcystein liegen bei ca. 1: 100, bevorzugt 1 : 50 oder 1 : 10.

Die erfindungsgemäß eingesetzten Wirkstoffe sind an sich bekannt. Sie können käuflich erworben oder in an sich bekannter Weise hergestellt und in physiologisch akzeptabler Weise formuliert werden.

Acetylcystein ist das N-Acetylderivat der natürlich vorkommenden Aminosäure L-Cystein. Da Acetylcystein von vielen Patienten bezüglich Geruch und Geschmack als unangenehm empfunden wird, wird es häufig in Form seiner Salze, bevorzugt seiner Alkalisalze, hier insbesondere bevorzugt als Natriumsalz, verabreicht. Weiterhin kann es in Form des Lysinats vorliegen (N-Acetylcystein-L-lysinat).

"Folsäure" bezeichnet erfindungsgemäss N-Pteroylglutaminsäure, die hierunter fallenden optischen Isomere sowohl als Gemische, z. B. als Racemat, als auch in Reinform, z. B. R-oder S-Enantiomere, eingeschlossen. Bevorzugt ist N-Pteroyl-L-glutaminsäure.

Zu den Folsäure-Derivaten gehören vor allem Folsäure-Metabolite sowie Amide und Ester der Folsäure wie auch der Metabolite. Vorteilhaft sind Amide und Ester, die unter physiologischen Bedingungen hydrolisierbar sind, wie Amide mit C1-Clo-Alkylaminen oder Ester mit C₁-C₁₀₋Alkoholen. Eine besondere Form der Amide sind N-Pteroylpoly-glutaminsäuren.

Die hierunter fallenden optischen Isomere sind entsprechend eingeschlossen, wobei auch hier die L-Glutaminsäure-Derivate bevorzugt sind. Insbesondere sind Tetrahydrofolsäure, 5-Methyltetrahydrofolsäure, 5,10-Methylentetrahydrofolsäure, 5-Formyltetrahydrofolsäure, 10-Formyltetrahydrofolsäure, 5-Formiminotetrahydrofolsäure und 5,10-Methenyltetrahydrofolsäure zu nennen.

Zu physiologisch akzeptablen Salzen von Folsäure bzw. Folsäure-Derivaten gehören Säure- und Basenadditionssalze sowie entsprechende Mischformen.

Zu den Säureadditionssalzen zählen Salze von Folsäure bzw. Folsäure-Derivaten mit anorganischen Säuren, wie Salzsäure, Schwefelsäure, Salpetersäure oder Phosphorsäure, oder organischen Säuren, insbesondere Carbonsäuren, z. B. Essigsäure, Weinsäure, Milchsäure, Citronensäure, Apfelsäure, Mandelsäure, Ascorbinsäure, Maleinsäure, Fumarsäure, Gluconsäure oder Sulfonsäuren, z. B. Methansulfonsäure, Benzolsulfon-säure und Toluolsulfonsäure, und dergleichen.

Zu den Basenadditionssalzen zählen Salze von Folsäure bzw. Folsäure-Derivaten mit anorganischen Basen, beispielsweise Metallhydroxiden bzw. -carbonaten von Alkali-, Erdalkali- oder Übergangsmetallen, oder mit organischen Basen, beispielsweise Ammoniak oder basischen Aminosäuren, wie Arginin und Lysin, Aminen, z. B. Methylamin, Dimethylamin, Trimethylamin, Triethylamin, Ethylamin, Diethylamin, Ethylendiamin, Ethanolamin, Diethanolamin,1-Amino-2-propanol, 3-Amino-1-propanol oder Hexamethylentetramin, gesättigten cyclischen Aminen mit 4 bis 6 Ringkohlenstoffatomen, wie Piperidin, Piperazin, Pyrrolidin und Morpholin, sowie weiteren organischen Basen, beispielsweise N-Methylglucamin, Kreatin und Tromethamin, sowie quaternären Ammoniumverbindungen, wie Tetramethylammonium und dergleichen.

Bevorzugt sind Salze mit anorganischen Basen, z. B. Na-, K-, Mg-, Ca-, Zn-, Cr-und Fe-Folate.

"Vitamin B6" bezeichnet erfindungsgemäss 4,5-Bis (Hydroxymethyl) -2-methyl-3-pyridinol, auch als Pyridoxin (INN) bezeichnet.

Zu den Vitamin B6-Derivaten gehören vor allem Pyridoxale, Pyridoxole und Pyridoxamine sowie Ester der Pyridoxine, Pyridoxole, Pyridoxale und Pyridoxamine. Vorteilhaft sind auch hier Ester, die unter physiologischen Bedingungen hydrolisierbar sind.

Insbesondere zu nennen sind in diesem Zusammenhang die Pyridoxine, Pyridoxale und Pyridoxamine.

Zu physiologisch akzeptablen Salzen von Vitamin B6 bzw. Vitamin B6-Derivaten gehören insbesondere Säureadditionssalze, z. B. mit den oben genannten anorganischen und organischen Säuren. Insbesondere sind die Hydrochloride, vor allem Pyridoxin-HCl, Ascorbate, Oxoglutarate und Phosphoserinate zu nennen.

"Vitamin B12"wird auch als Cyanocobalamin oder Cobalamin bezeichnet.

Zu den Vitamin-B12-Derivaten gehören insbesondere Cobalamine, in denen die Cyanogruppe des Cyanocobalamins durch andere Koordina- tionspartner des Cobalts ersetzt ist. Hierzu zählen vor allem das Hydroxocobalamin, Aquocobalamin, Nitrosocobalamin, Methylcobalamin und Adenosylcobalamin (Coenzym B12).

Zu physiologisch akzeptablen Salzen von Vitamin B 12 bzw. Vitamin B 12-Derivaten gehören insbesondere Säureadditionssalze z. B. mit den oben genannten anorganischen und organischen Säuren. Insbesondere ist das Acetat des Hydoxocobalamins zu nennen.

Zu den Vorstufen gehören alle Verbindungen, die in vivo zu den biologisch aktiven Wirkstoffen der erfindungsgemäßen Zubereitung umgewandelt werden.

Assays zur Bestimmung von Homocysteinspiegeln, die normalerweise in einem Bereich von 5 bis 13,9 µmol/l Blutplasma liegen, sind bekannt (vgl. z. B. den eingangs geschilderten Stand der Technik).

Erhöhte Homocysteinspiegel werden als Hyperhomocysteinämie bezeichnet. Mithilfe der erfindungsgemässen Mittel können erhöhte Homocysteinspiegel gesenkt bzw. präventiv vermieden werden.

Je nach Homocysteinspiegel, werden Hyperhomocysteinämien in Klassen eingeteilt : Eine moderate Hyperhomocysteinämie ist durch Nüchtern-Hcy von 10-30 µmol/l gekennzeichnet. Eine schwere ist Hyperhomocysteinämie gekennzeichnet mit Nüchtern-Hcy Werten > 30 µmol/l.
Bei Gesunden sind Homocysteinwerte von 10-12 µmol/l tolerabel.

Besonders bevorzugt wird die erfindungsgemäße Kombination aus Wirkstoffen zur Therapie eines erhöhten Homocysteinserumspiegels spezielle bei Patienten in der Prädialyse oder Dialyse eingesetzt, zur Therapie von Patienten mit einer gestörten Transsulphurierung, der Behandlung von Durchblutungsstörungen, Vitamin-Mangelzuständen, peripheren Neuropathien sowie Kachexien. Selbstverständlich kann die erfindungsgemäße Kombination an Wirkstoffen, enthaltend zumindest Vitamin B6 und Acetylcystein, zur Behandlung aller anderen Erkrankungen eingesetzt werden, die mit erhöhten Homocysteinspiegeln in Verbindung stehen, insbesondere damit einhergehen oder dadurch verursacht werden.

Erhöhte Homocysteinspiegel sind insbesondere bei Dialysepatienten zu beobachten. Hierzu gehören die Prädialysepatienten, besonders aber die Dialysepatienten mit einer Hyperhomocysteinämie und gestörter Transsulphurierung. Aber auch bei nicht Dialysepatienten sind erhöhte Homocysteinspiegel zu beobachten und Auslöser bzw. stehen in engem Zusammenhang mit vaskulären Erkrankungen der peripheren, koronaren und zerebralen Gefäße. Hierzu gehören Veränderungen an den Gefäßendothelzellen, eine Proliferation der Muskelzellen und/oder eine Verdickung der Gefäßintema. Behandelbar sind deshalb insbesondere Atherosklerosen, venöse Thrombosen, arterielle Verschlüsse und andere arteriovenöse Gefäßleiden.

Die Behandlung erfolgt insbesondere bevorzugt in den oben angegebenen Mengenverhältnissen. Der Fachmann, hier ein Humanmediziner bzw. ein Phannazeut, können in Abhängigkeit vom Krankheitsbild, vom zu behandelnden Patienten und weiteren an sich bekannten Kriterien die vorteilhafte und therapeutisch wirksame Dosis und die Art und Weise der Applikation unter Berücksichtigung von Kontrollunter-suchungen, bestimmen. Beispielsweise sind insbesondere bei Prädialysepatienten orale Darreichungsformen bevorzugt, während beim Dialysepatienten häufig intravenöse Applikationsformen gewählt werden. Die pharmazeutische Zusammensetzung der Erfindung wird deshalb insbesondere als orale und parenterale Darreichungsform eingesetzt.

Die Formulierungsgrundlage der erfindungsgemäß bereitgestellten pharmazeutischen Zusammensetzungen umfaßt pharmazeutisch übliche Trägerstoffe und/oder Hilfsstoffe. Physiologisch verträglich sind im Bereich der Pharmazie, der Lebensmitteltechnologie und den angrenzenden Gebieten üblicherweise eingesetzten Hilfsstoffe, wie sie in den Arzneibüchern, beispielsweise DAB, der US Pharmacopeia usw., enthalten sind.

Geeignete Hilfsstoffe können sein: Netzmittel; emulgierende und suspendierende Mittel; konservierende Mittel; Antioxidantien; Antireizstoffe; Chelatbildner; Dragierhilfsmittel; Emulsionsstabilisatoren; Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe; wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Beispiele geeigneter pharmazeutischer Formulierungen sind feste Arzneiformen, wie Pulver, Puder, Granulate, Tabletten, insbesondere Filmtabletten, Pastillen, Sachets, Cachets, Dragees, Kapseln wie Hart- und Weichgelatinekapseln, sowie flüssige Arzneiformen, wie Lösungen, Emulsionen, insbesondere Öl-in-Wasser-Emulsionen, Suspensionen, beispielsweise Lotionen, Injektions-und Infusionszubereitungen. Auch implantierte Abgabevorrichtungen können zur Verabreichung erfindungsgemässer Wirkstoffe verwendet werden. Ferner können auch Liposomen oder Mikrosphären zur Anwendung kommen.

Lebensmitteltechnische Formulierungen haben in der Regel die übliche Form und werden bevorzugt in Form von Kleinkindnahrung, Frühstückszubereitungen, vor allem in Form von Müslis oder Riegeln, Sportlerdrinks, Komplettmahlzeiten, insbesondere im Rahmen von Diäten, diätetischen Zubereitungen, wie Diätdrinks, Diätmahlzeiten und Diätriegel, angeboten.

Die Formulierungen werden vorzugsweise auf oralem, sie können aber auch insbesondere im Bereich der Arzneimittel auf intravenösem oder intramuskulärem Weg verabreicht werden.

Bei der Herstellung der Zusammensetzungen, die in an sich bekannter Weise erfolgen kann, werden die Wirkstoffe gewöhnlich mit einem geeigneten Hilfsstoff, in diesem Fall auch als Exzipient bezeichnet, vermischt oder verdünnt. Exzipienten können feste, halbfeste oder flüssige Materialien sein, die als Vehikel, Träger oder Medium für den Wirkstoff dienen. Die Zumischung weiterer Hilfsstoffe erfolgt erforderlichenfalls in an sich bekannter Weise. Es können Formgebungsschritte, gegebenenfalls in Verbindung mit Mischvorgängen, durchgeführt werden, z. B. eine Granulierung, Komprimierung und ähnliches.

Insbesondere können die Wirkstoffkomponenten in an sich bekannter Weise gemeinsam formuliert werden. Sie können aber auch zunächst getrennt verarbeitet und anschliessend in einer kompar-timentierten, z. B. mehrschichtigen Arzneiform zusammengeführt werden. Dadurch kann möglichen Wirkstoffinkompatibilitäten und unterschiedlichen Wirkstoffeigenschaften, wie Bioverfügbarkeit, Stabilität, Löslichkeit und ähnlichem, Rechnung getragen werden.

Literatur
1. Bachmann J, Tepel M, Raidt H, Riezler R, Graefe U, Langer K, Zidek W: Hyperhomocysteinemia and the risk for vascular disease in hemodialysis patients. J Am Soc Nephrol 6:121-125,1995
2. Herrmann W, Schorr H, Geisel J, Riegel W. Homocysteine, Cystathionine, Methylmalonic Acid and B-Vitamins in Patients with Renal Disease. Clin Chem Lab Med 2001;39(8):739-746.
3. Wald DS, Law M, Morris JK. Homocysteine and cardiovascular disease: evidence on causality from a meta-analysis. BMJ 2002;325(7374):1202.
4. Van Guldener C, Stam F, Stehouwer CDA: Homocysteine metabolism in renal failure. Kidney Int 59:S234-S237,2001
5. Friedman AN, Bostom AG, Selhub J, Levey AS, Rosenberg IH: The kidney and homocysteine metabolism. J Am Soc Nephrol 12:2181-2189,2001
6. Loscalzo J: The oxidant stress of hyperhomocysteinemia. J Clin Invest 98:5-7,1996
7. Harper PC, Chang VT, Borth W: homocysteine and other sulfhydryl compounds enhance the binding of lipopotein(a) to fibrin: A potential biochemical link between thrombosis, atherogenesis, and sulfhydryl compound metabolism. Roc Natl Acad Sci USA 89:10193-10197,1992
8. Bostom AG, Culleton BF: Hyperhomocysteinemia in chronic renal failure. J Am Soc Nephrol 10:891-900,1999
9. Shemin D, Bostom AG, Selhub J. Treatment of hyperhomocysteinemia in end-stage renal disease. Am J Kidney Dis. 2001 Oct;38(4 Suppl 1):S91-4.
10. Henning BF, Zidek W, Riezler R, Graefe U, Tepel M. Homocyst(e)ine metabolism in hemodialysis patients treated with vitamins B6, B12 and folate. Res Exp Med (Berl). 2001 Mar;200(3):155-68.
11. Dierkes J, Domrose U, Ambrosch A, Schneede J, Guttormsen AB, Neumann KH, Luley C. Supplementation with vitamin B12 decreases homocysteine and methylmalonic acid but also serum folate in patients with end-stage renal disease. Metabolism 48:631-635,1999.
12. Bostom AG, Shemin D, Lapane KL, Hume AL, Yobum D, Nadeau MR, Bendich A, Selhub J, Rosenberg ICH: High dose B-vitamin treatment of hyperhomocysteinemia in dialysis patients. Kidney Int 49:147-152,1996
13. Hultberg B, Andersson A, Masson P, Larson M, Tunek A: Plasma homocysteine and thio compound fractions after oral administration of N-acetylcysteine. Scand J Clin Lab Invest 54:417-423,1994
14. Friedman AN, Bostom AG, Laliberty P, Selhub J, Shemin D. The effect of N-acetylcysteine on plasma total homocysteine levels in hemodialysis: A randomized, controlled study. Am J Kidney Dis 2003;41:442-6.
15. Peterson JC, Spence JD: Vitamins and progression of atherosclerosis in hyper-homocyst(e)inaemia [Letter]. Lancet 1998;351: 263
16. Naurath HJ, Riezler R, Pütter S, Ubbink JB. Does a Single Vitamin B-Supplementation Induce Functional Vitamin B-Deficiency. Clin Chem Lab Med. 2001; 39:768-771
17. Brattstrom L, Lindgren A, Israelsson B, Andersson A, Hultberg B: Homocysteine and cysteine: Determinants ofplasma levels in middle-aged and elderly subjects. J Intern Med 1994;236: 633-641
18. Hermann, V. et al.
   Clin. Chem. Lab. Med. 2001, 39(8), 739 - 746

## Patentansprüche

1. Pharmazeutische Zusammensetzung, enthaltend eine therapeutisch wirksame Menge an Vitamin B6 und Acetylcystein, und/oder ihrer physiologisch akzeptablen Derivate und/oder ihrer Vorstufen und/oder ihrer pharmazeutisch wirksamen Salze, zusammen mit pharmazeutisch üblichen Träger- und/oder Hilfsstoffen.

2. Pharmazeutische Zusammensetzung nach Anspruch 1, wobei Vitamin B6 in einer Menge vorliegt, so dass 0,1 - 500 mg, bevorzugt 5 - 300 mg, weiterhin bevorzugt 5 - 200 mg oder 0,1 - 150 mg oder 0,1 -100 mg pro Tag verabreicht werden, und/oder Acetylcystein in einer Menge vorliegt, so dass 0,1 - 5000 mg, bevorzugt 0,1 - 4000 mg, weiterhin bevorzugt 0,1 - 3600 mg oder 0,1 - 1800 mg pro Tag verabreicht werden.

3. Pharmazeutische Zusammensetzung nach Anspruch 1 oder 2, wobei Vitamin B6 und Acetylcystein in einer Mengenverhältnis von 1 : 100, bevorzugt 1 : 50 oder 1 : 10 vorliegen.

4. Pharmazeutische Zusammensetzung nach einem oder mehreren der vorhergehenden Ansprüche, wobei als weitere Wirkstoffe Vitamin B 12 und/oder Folsäure enthalten sind.

5. Pharmazeutische Zusammensetzung nach Anspruch 4, wobei Vitamin B 12 in einer Menge von 0,01 - 3 mg, bevorzugt 0,01 - 1,0 mg und weiterhin bevorzugt 2 - 3 mg/Tag verabreicht wird, und/oder Folsäure in einer Menge von 0,1 - 30 mg/Tag, bevorzugt 0,1 - 25 mg und weiterhin bevorzugt 25 - 30 mg/Tag verabreicht wird.

6. Pharmazeutische Zusammensetzung nach einem oder mehreren der vorhergehenden Ansprüche, wobei die Wirkstoffe in einer Applikationsdosis oder getrennt von einander vorliegen.

7. Pharmazeutische Zusammensetzung nach einem oder mehreren der vorhergehenden Ansprüche, wobei als Salze von Acetylcystein die Alkalisalze, bevorzugt Na, als Salze von Vitamin B6 das Acetat oder das Phosphat, als Salze der Folsäure das Mono-Natriumsalz oder das Calciumsalz und als Salze des Vitamins B 12 das Acetat oder Phosphat eingesetzt werden.

8. Pharmazeutische Zusammensetzung nach einem oder mehreren der vorhergehenden Ansprüche, wobei sie als orale oder parenterale Darreichungsform vorliegt.

9. Verfahren zur Herstellung einer pharmazeutischen Zusammensetzung nach einem oder mehreren der vorhergehenden Ansprüche, wobei Vitamin B6 und Acetylcystein und/oder Vitamin B12 und/oder Folsäure und/oder ihre Vorstufen und/oder ihre pharmazeutisch wirksamen Salze in einer therapeutisch wirksamen Menge, zusammen mit den pharmazeutisch üblichen Träger- und/oder Hilfsstoffen, vermischt und zu einer pharmazeutisch wirksamen Zusammensetzung formuliert werden.

10. Verwendung einer therapeutisch wirksame Menge an Vitamin B6 und Acetylcystein und/oder Vitamin B12 und/oder Folsäure und/oder ihrer Vorstufen und/oder ihrer pharmazeutisch wirksamen Salze, zusammen mit pharmazeutisch üblichen Träger- und/oder Hilfsstoffen, nach einem oder mehreren der vorhergehenden Ansprüche zur Herstellung einer pharmazeutischen Zusammensetzung zur Therapie und Prophylaxe erhöhter Homocysteinwerte, und zur Absenkung der Cystathionin- und Cysteinspiegel, insbesondere bei Dialysepatienten und Patienten mit gestörter Transsulphurierung, von Vitaminmangelzuständen, Durchblutungsstörungen, kardio- und zerebrovaskulären Erkrankungen, Neuropathien und Kachexien.
